# EUROPEAN PATENT APPLICATION

(11) **EP 4 424 262 A1**
(43) Date of publication of application: **04.09.2024**
(21) Application number: 24155527.5
(22) Date of filing: 02.02.2024
(51) Int. Cl.: A61B 34/10, A61B 5/00, A61B 5/055, A61B 5/20, A61B 18/00

(54) **SELECTION OF DENERVATION THERAPY**

(30) Priority: 28.02.2023 US 202363487398 P
(71) Applicant: Medtronic Ireland Manufacturing Unlimited Company, Dublin 2, D02 T380 (IE)
(72) Inventor: Lima, Carlos H., Santa Rosa, 95403 (US); Bliss, Richard J., Santa Rosa, 95403 (US); Tunev, Stefan S., Santa Rosa, 95403 (US)
(74) Representative: Zimmermann & Partner Patentanwälte mbB

(57) **Abstract**

An example computing device includes a memory; and processing circuitry coupled to the memory, the processing circuitry being configured to: receive renal arterial anatomy information of a patient; determine one or more of a location of renal denervation therapy or a modality of renal denervation therapy to apply to the patient based on the renal arterial anatomy information; and output an indication of one or more of the determined location to apply renal denervation therapy to the patient or the determined modality of renal denervation therapy to apply to the patient.

## Description

### TECHNICAL FIELD

This disclosure generally relates to denervation therapy.

### BACKGROUND

Percutaneous renal denervation is a procedure that can be used for treating hypertension. During a renal denervation procedure, a clinician delivers stimuli or energy, such as radiofrequency, ultrasound, cooling or other energy, to a treatment site to reduce activity of nerves surrounding a blood vessel. The stimuli or energy delivered to the treatment site may provide various therapeutic effects through alteration of sympathetic nerve activity. Percutaneous denervation of the afferent and efferent renal nerves may result in blood pressure (BP) lowering in some patients with uncontrolled hypertension in both the presence and absence of concomitant anti-hypertensive drug therapy.

### SUMMARY

Denervation therapy may provide a therapeutic benefit to certain patients. For example, renal denervation (RDN) may mitigate symptoms associated with renal sympathetic nerve overactivity. However, there is difficulty determining what modality and/or location of RDN therapy is most suitable for a particular patient to produce the best results. Various aspects of the techniques described in this disclosure relate to a computing device configured to determine one or more of a modality of RDN therapy to apply to the patient or a location to apply RDN therapy based on the renal arterial anatomy information of the patient who will undergo the RDN procedure. As such, the various aspects of the techniques may reduce a number of ablations being performed while achieving a desired result for the patient, which in turn improves health care efficiency, while also reducing patient discomfort in undergoing an RDN procedure to deliver denervation therapy. Renal arterial anatomy information for a respective kidney may be obtained via an aortogram, CT scan, or other imaging devices.

Accordingly, the present disclosure describes a predictor to determine, before performing an RDN procedure to deliver denervation therapy, which location and/or modality of renal denervation therapy should achieve a desired RDN therapy result in a patient, while reducing unnecessary denervation. This determination may help identify one or more location, such a location of a main renal artery, to deliver a particular modality of RDN therapy to achieve a desired RDN therapy result for a particular patient. For instance, a computing device may determine a location and/or modality of renal denervation therapy before performing an invasive denervation procedure, which may improve health care efficiency and patient care by improving performance of procedures, reducing performance of ineffective or unnecessary procedures (e.g., performing simpler RDN procedure using determined location and/or modality of RDN therapy) and/or reducing patient discomfort from undergoing an unnecessary complex procedures (e.g., performing a complex RDN procedure on a patient to produce results that may be accomplished by performing simpler RDN procedure using determined location and/or modality of RDN therapy).

In one example, this disclosure is directed to a computing device comprising: a memory; and processing circuitry coupled to the memory, the processing circuitry being configured to: receive renal arterial anatomy information of a patient; determine one or more of a location of renal denervation therapy or a modality of renal denervation therapy to apply to the patient based on the renal arterial anatomy information; and output an indication of one or more of the determined location to apply renal denervation therapy to the patient or the determined modality of renal denervation therapy to apply to the patient.

In another example, this disclosure is directed to a system comprising: an imaging device configured to collect data indicative of renal arterial anatomy information of a patient; and a computing device communicatively coupled to the imaging device, wherein the computing device comprises: a memory; and processing circuitry coupled to the memory, the processing circuitry being configured to: receive the renal arterial anatomy information of the patient; determine one or more of a location of renal denervation therapy or a modality of renal denervation therapy to apply to the patient based on the renal arterial anatomy information; and output an indication of one or more of the determined location to apply renal denervation therapy to the patient or the determined modality of renal denervation therapy to apply to the patient.

In another example, this disclosure is directed to a method comprising: receiving renal arterial anatomy information of a patient; determining one or more of a location of renal denervation therapy or a modality of renal denervation therapy to apply to the patient based on the renal arterial anatomy information; and outputting an indication of one or more of the determined location to apply renal denervation therapy to the patient or the determined modality of renal denervation therapy to apply to the patient.

Further disclosed herein is a computing device that includes a memory; and processing circuitry coupled to the memory, the processing circuitry being configured to: receive renal arterial anatomy information of a patient; determine one or more of a location of renal denervation therapy or a modality of renal denervation therapy to apply to the patient based on the renal arterial anatomy information; and output an indication of one or more of the determined location to apply renal denervation therapy to the patient or the determined modality of renal denervation therapy to apply to the patient.

Further details of one or more examples of this disclosure are set forth in the accompanying drawings and in the description below. Other features, objects, and advantages will be apparent from the description and drawings, and from the claims.

The above summary is not intended to describe each illustrated example or every implementation of the present disclosure.

### BRIEF DESCRIPTION OF DRAWINGS

FIG. 1 is a conceptual diagram illustrating an example system for determining a modality and/or location of denervation therapy to apply to a patient, in accordance with some examples of the current disclosure.
FIG. 2 is a block diagram illustrating an example configuration of a computing device and an imaging device, in accordance with some examples of the current disclosure.
FIGS. 3A-3B are conceptual diagrams of renal arterial anatomy information accordance with some examples of the current disclosure.
FIG. 3C is a conceptual diagram of an image of a main renal artery to be displayed that indicates a location to apply renal denervation therapy, in accordance with some examples of the current disclosure.
FIG. 4 is a conceptual diagram illustrating an example neural network configured to determine one or more of a location or modality of renal denervation therapy to apply to a patient.
FIG. 5 is a flow diagram illustrating an example technique for operating a system to determine one or more of a location or modality of renal denervation therapy to apply to a patient.
FIG. 6 is a flow diagram illustrating an example technique for operating a system to determine a location of renal denervation therapy to apply to a patient.

### DETAILED DESCRIPTION

Denervation therapy, such as renal denervation (RDN) therapy, may be used to render a nerve inert, inactive, or otherwise completely or partially reduced in function, such as by ablation or lesioning of the nerve. Following denervation, there may be a reduction or even prevention of neural signal transmission along the target nerve. Denervating an overactive nerve may provide a therapeutic benefit to a patient. For example, renal denervation may mitigate symptoms associated with renal sympathetic nerve overactivity, such as hypertension. Denervation therapy may include delivering electrical and/or thermal energy to a target nerve, and/or delivering a chemical agent to a target nerve. In the case of renal denervation therapy, the denervation energy or chemical agents can be delivered, for example, via a therapy delivery device (e.g., a catheter) disposed in a blood vessel (e.g., the main renal artery) proximate to the renal nerve.

The renal sympathetic nervous system has been identified as a contributor to the complex pathophysiology of hypertension, or elevated systemic blood pressure (BP). Therefore, renal denervation may reduce renal sympathetic nerve overactivity and cause a reduction in systemic BP as a treatment for hypertension. In some patients, renal denervation may reduce systolic BP in a range of approximately 5 millimeters of mercury (mmHg) to 30 mmHg. However, renal denervation may not reduce systolic BP for other patients. RDN therapy may be used as treatment for other conditions associated with changes in sympathetic nervous system activity as well, such as arrhythmias and heart failure.

RDN modalities may include modalities such as radiofrequency, cryoablation, ultrasound, microwave, chemical, radiation, nanoparticles, pulse field ablation, and other modalities. In addition, there are many locations within the renal arteries in which RDN therapy may be applied. One approach for RDN therapy is to ablate at as many locations within the renal artery 'tree' as possible within the allowable range of vessel diameter and location of ablation site relative to the kidney. Although this approach has been shown to be safe and efficacious, some of these ablations may be unnecessary for some patients, e.g., based on proximity of renal nerves to the renal arteries. Performing unnecessary ablations may cause unnecessarily longer RDN procedures, unnecessarily higher costs of RDN therapy, unnecessary patient inconvenience and discomfort, and/or unnecessary potential side effects. In some examples, performing a single RDN therapy procedure at the main renal artery may be desirable, but determining an effective location at which to apply RDN therapy in the main renal artery that produces effective results has been difficult as an effective RDN therapy location may differ depending on the patient.

In some examples, a main renal artery may be a blood vessel that feeds the largest volume of blood from the aorta to a respective kidney.

In some examples, there may be more than one blood vessel that feeds blood from the aorta to the respective kidney. For example, if two or more blood vessels each feed a similar amount of volume of blood to the respective kidney, and those respective two or more blood vessels each respectively feed the largest volume of blood to the respective kidney, and each respectively feed a volume of blood from the aorta to the respective kidney above a main renal artery volume threshold, those two or more blood vessels that respectively feed the largest volume of blood to the respective kidney may each be identified as a respective main renal artery.

In some examples, when there are additional blood vessels that feed blood from the aorta to the kidney, but feed a volume of blood lower than a volume of blood fed by the main renal artery, and the volume of blood being fed by the additional blood vessels being below a main renal artery volume threshold, those particular additional blood vessels may be identified as accessory renal arteries. In some examples, accessory arteries may originate from the aorta or vessels distal of the aorta such as the iliac artery. In some examples, accessory arteries may originate superior or inferior to the origin of the main renal artery.

In some examples, a length of a main renal artery may refer to a portion of a renal blood vessel originating from the aorta to a first or primary bifurcation point. In some examples, a portion of the main renal artery from the aorta or main renal artery ostium (typically located at the aorta) to a first or primary bifurcation point may be referred to as a "trunk" of that particular main renal artery, while the portions of the blood vessel past the first or primary bifurcation point may be referred to as "branches" of the main renal artery.

In some examples of the present disclosure, processing circuitry (e.g., of a computing device) may be configured to determine an ablation strategy based on the renal arterial anatomy. Processing circuitry may be configured to determine one or more of a modality of RDN therapy to apply to the patient or a location to apply RDN therapy based on the renal arterial anatomy information. While the above examples discuss renal denervation responsiveness in reducing hypertension, the techniques described herein may similarly be used to determine renal denervation responsiveness for treatment of other conditions associated with changes in sympathetic nervous system activity, such as arrhythmias and heart failure.

A computing device or system may be configured receive renal arterial anatomy information for a patient. In some examples, renal arterial anatomy may be received via an aortogram, angiogram, computerized tomography (CT) scan, or other devices. The computing device or system may be configured to determine one or more of a modality of RDN therapy to apply to the patient or a location to apply RDN therapy based on the renal arterial anatomy information of the patient who will undergo the RDN procedure. In some examples, rather than or in addition to being configured to determine the modality of the RDN therapy to apply based on the renal arterial anatomy information, the computing device or system may be configured to receive information indicative of a modality of renal denervation therapy that was selected to apply to the patient. In some such examples, the computing device or system may be configured to determine the location of renal denervation therapy based on the renal arterial anatomy information and the selected modality of renal denervation therapy, and output an indication of the determined location to apply the renal denervation therapy to the patient.

Determining a patient specifc targeted ablation strategy, such as determining one or more of a modality of RDN therapy or location(s) of RDN therapy, before performing a denervation procedure, in accordance with various techniques of this disclosure, may produce improved results and may reduce unnecessary ablations (e.g., ineffective modality and/or ineffective location) on patients that may not sufficiently help achieve a desired clinical result. This may help reduce unnecessary health care costs by reducing RDN procedure time in some patients while also reducing unnecessary discomfort by reducing unnecessary ablations.

While the above examples discuss determining a patient specific targeted ablation strategy based on the renal arterial anatomy of the patient who will undergo the RDN procedure, the techniques described herein may similarly be used to determine a denervation strategy based on arterial anatomy at other locations, such as at or around a pulmonary artery, hepatic artery, celiac artery, mesenteric artery, arteries that feed a digestive system, arteries that feed a reproductive system, and/or other arteries. In some examples, the techniques described herein may similarly be used, based on arterial anatomy at multiple locations, to determine a denervation strategy at multiple locations, such as two or more of a main renal artery, a pulmonary artery, hepatic artery, celiac artery, mesenteric artery, arteries that feed a digestive system, arteries that feed a reproductive system, and/or other arteries.

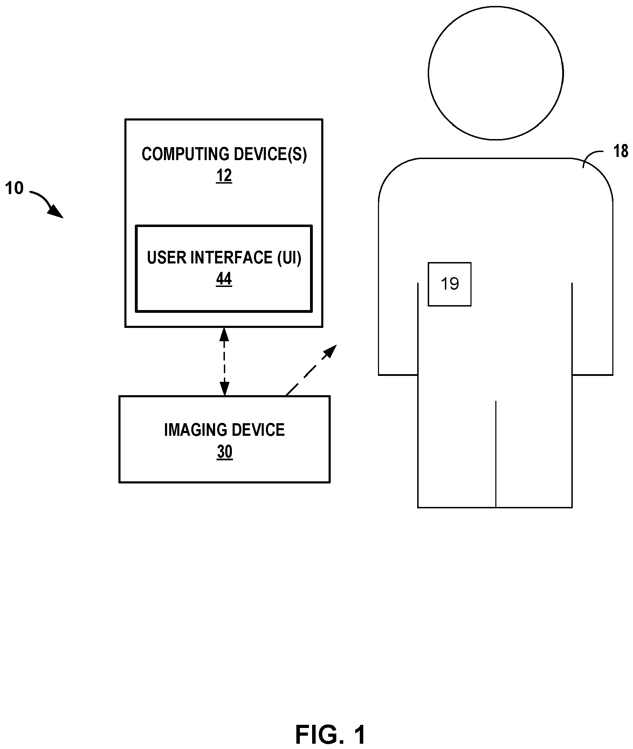

FIG. 1 is a conceptual diagram illustrating an example system 10 for determining a location and/or modality of RDN therapy to apply to a patient, in accordance with some examples of the current disclosure. As shown in FIG. 1, system 10 includes a computing device 12. Computing device 12 may be a computing device used in a home, ambulatory, clinic, or hospital setting. Computing device 44 may include, for example, a clinician programmer, a desktop computer, a laptop computer, a workstation, a server, a mainframe, a cloud computing system, a smartphone, combinations thereof, or the like. Computing device 12 may be configured to receive, via a user interface device 44 ("UI 44"), input from a user, such as a clinician; output information to a user; or both. In some examples, UI 44 may include a display (e.g., a liquid crystal display (LCD) or light emitting diode (LED) display), such as a touch-sensitive display; one or more buttons; one or more keys (e.g., a keyboard); a mouse; one or more dials; one or more switches; a speaker; one or more lights; combinations thereof; or the like. Imaging device 30 may be an imaging modality that may collect imaging data of anatomy of patient 18, such as arterial anatomy. In some examples, imaging device 30 may collect imaging data of renal arterial anatomy 19 of patient 18. Computing device 12 may receive the collected imaging data and determine a location and/or modality of RDN therapy to apply based, at least in part, on the collected imaging data.

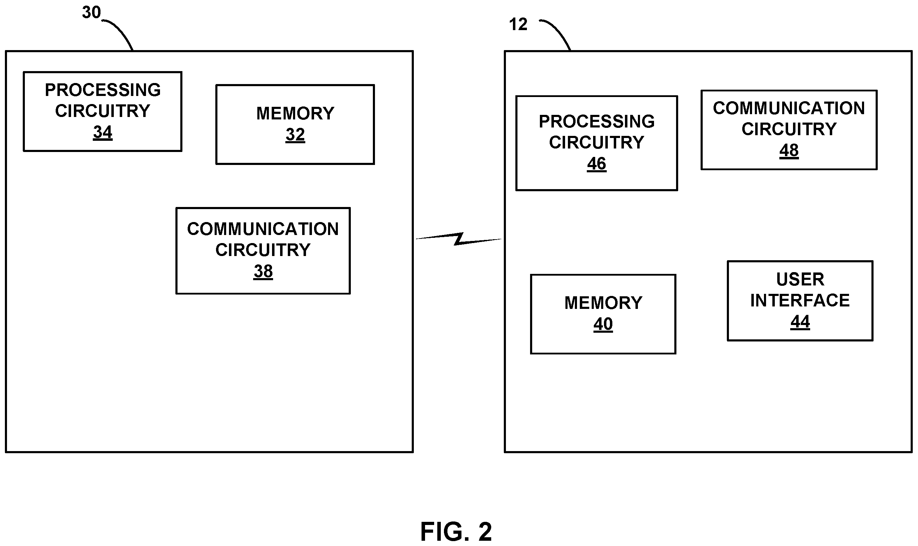

FIG. 2 is a block diagram illustrating an example configuration of computing device 12 of FIG. 1 and imaging device 30. As shown in FIG. 2, imaging device 30 may include processing circuitry 34, memory 32, and communication circuitry 38. As shown in FIG. 2, computing device 12 may include processing circuitry 46, communication circuitry 48, memory 40, and UI 44. Memory 32 or 40 may include any volatile or non-volatile media, such as a random access memory (RAM), read only memory (ROM), non-volatile RAM (NVRAM), electrically erasable programmable ROM (EEPROM), flash memory, or the like. Memory 32 or 40 may store computer-readable instructions that, when executed by processing circuitry 34 or 46, respectively, cause imaging device 30 or computing device 12, respectively, to perform various functions described herein. Processing circuitry 34 or 46 may include any combination of one or more processors including one or more microprocessors, digital signal processors (DSPs), application specific integrated circuits (ASICs), field programmable gate arrays (FPGAs), or other equivalent integrated or discrete logic circuitry. Accordingly, processing circuitry 34 or 46 may include any suitable structure, whether in hardware, software, firmware, or any combination thereof, to perform the functions ascribed herein to processing circuitry 34 or 46. Computing device 12 and imaging device 30 may be configured to receive data (e.g., via communication circuitry 48 or 38) from another computing device. In some examples, computing device 12 and imaging device 30 may be separate devices. In some examples, computing device 12 and imaging device 30 may be located on the same device.

Computing device 12 may receive arterial anatomy information of patient 18. Examples of arterial anatomy information of patient 18 include images and/or imaging data of arterial anatomy. Other examples of arterial anatomy information of patient 18 may include textual information indicative of relative locations of structures within the arterial anatomy, etc.
Some examples of arterial anatomy information may include one or more of length of main renal artery, diameter of main renal artery, artery wall thickness, artery wall stiffness, bifurcation location relative to aorta to hilum distance, relative height of renal take-off (for example, using vertebrae as measure of height), location where main renal artery enters kidney (number of access points away from renal hilum), arterial anatomy information may include one or more of accessory artery count or accessory artery take-off relative to main artery (inferior, superior, ostial separation distance), or ratios or differences in wall thickness and/or stiffness at different locations of main renal artery. In some examples, left and right kidneys may be evaluated independently or combined. For example, arterial anatomy information may include a ratio of right vs left main artery length.

In some examples, arterial anatomy information of patient 18 may include or further include one or more of length of main pulmonary, hepatic, or other artery, diameter of main pulmonary, hepatic, or other artery, main pulmonary, hepatic, or other artery wall thickness, main pulmonary, hepatic, or other artery wall stiffness, bifurcation location relative to pulmonary, hepatic, or other artery distance, relative height of main pulmonary, hepatic, or other artery take-off (for example, using vertebrae as measure of height), location where main pulmonary, hepatic, or other artery enters organ, ratios/differences in main pulmonary, hepatic, or other artery wall thickness at different locations, and/or ratios/differences of main pulmonary, hepatic, or other artery wall stiffness at different locations. Other examples of arterial anatomy information may include accessory artery count, accessory artery take-off relative to main pulmonary, hepatic, or other artery (inferior, superior, ostial separation distance).

In some examples, computing device 12 may receive renal arterial anatomy information for a respective kidney via an aortogram, angiogram, CT scan, or other imaging techniques. For instance, imaging device 30 may capture images or imaging data of the arterial anatomy, and transmit such images to computing device 12. In some examples, contrast may be released in an aorta of patient 18, which then flows downstream and feeds into the renal arteries, to obtain renal arterial anatomy information for a respective kidney via an aortogram or CT scan, which are examples of the images that imaging device 30 may capture and/or generate.

In some examples, computing device 12 may receive renal arterial anatomy information via another computing device, such as a server, storing renal arterial anatomy information of patient 18. In some examples, computing device 12 may store renal arterial anatomy information of patient 18. In some examples, imaging device 30 may collect renal arterial anatomy information of patient 18.

In some examples, imaging device 30 may store renal arterial anatomy information in memory 32. Imaging device 30 may be communicatively coupled to computing device 12 and may transmit collected renal arterial anatomy information to computing device 12 via communication circuitry 38, either directly or via other computing devices. In some examples, the renal arterial anatomy information of patient 18 may be received by computing device 12 before an RDN catheter is introduced into patient 18 to perform a denervation procedure.

Determining a patient specific targeted ablation strategy, such as determining one or more of a modality of RDN therapy or location of RDN therapy, before performing a denervation procedure may help produce improved RDN therapy results and may reduce unnecessary ablations (e.g., ineffective modality and/or ineffective location) on patients that may not sufficiently help achieve a desired clinical result. This may help reduce unnecessary health care costs by reducing RDN procedure time in some patients while also reducing unnecessary discomfort in a patient by reducing unnecessary ablations.

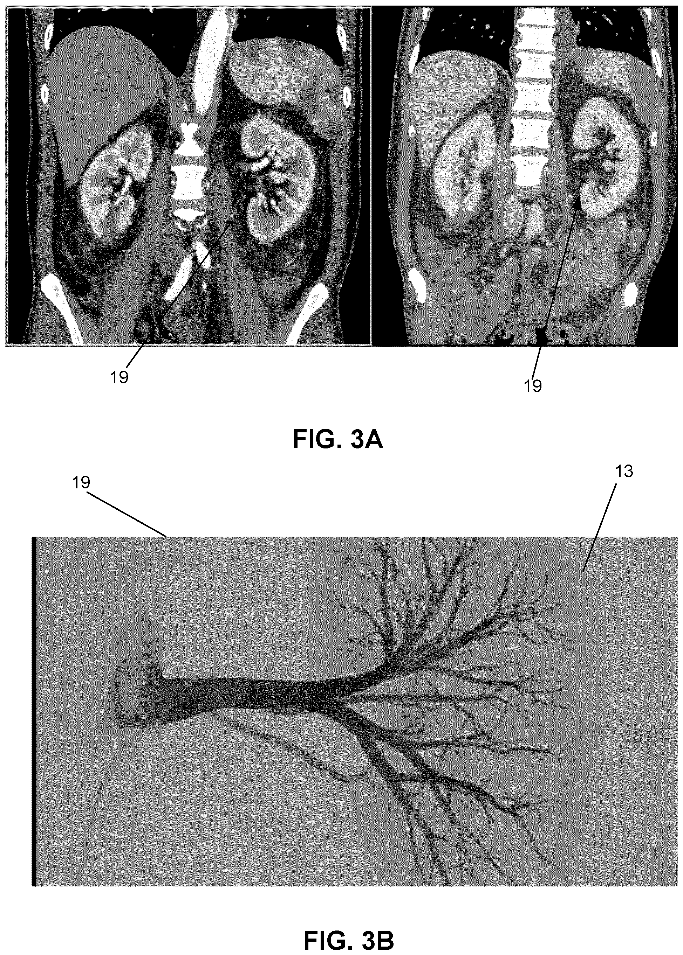

FIGS. 3A-3B show examples of renal arterial anatomy information 19 with respect to kidney 13. The arterial anatomy information may include one or more of a length of aorta to hilum, presence/numbers of non-renal branches (adrenal), number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, length of distal segment of the main renal artery, or angle of main renal artery and/or branches, or carinal position relative to a location of a feature of the main renal artery.

In some examples, processing circuitry 46 may determine a modality of RDN therapy to apply to a patient based on renal arterial anatomy information and output the determined modality. In other examples, a modality of RDN therapy to apply to a patient may be an input as processing circuitry 46 may determine a location of RDN therapy based, at least in part, on renal arterial anatomy information and the modality of RDN therapy.

Processing circuitry 46 may determine a location of RDN therapy to apply to patient 18 based on one or more of a length of space from aorta to renal hilum, number of branches, location of branches, length of main renal artery from aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery. For example, the length of the space from aorta to renal hilum may be divided in thirds and the two distal thirds be targeted by renal denervation. For example, RDN therapy may be modulated based on a minimum depth of ablation required to provide therapy while minimizing damage to other organs.

Locations of a renal nerve tend to be further away from portions of the main renal artery that are closer to the aorta. Renal nerves start converging toward the main renal artery and/or branches, the more distal a position gets from the aorta and the closer a position gets to the kidney. For example, processing circuitry 46 may determine to apply RDN therapy at a location based on a minimum depth of ablation required at that location and a minimum amount of potential damage to other tissue (e.g., organs). Apply RDN therapy at a location where the renal nerves converge that may require minimal ablation depth may improve effectiveness of the RDN therapy and/or reduce discomfort to patient during RDN therapy.

For example, RDN therapy may be applied, based on the anatomy of the patient, at a location deep enough that the renal nerves are converging to likely be ablated with a minimal amount of ablation depth, while not being too deep to potentially damage other tissue, such as other organs, not intended to receive the applied RDN therapy. In some examples, processing circuitry 46 may determine a location of RDN therapy to apply to patient that achieves the greatest balance of successful RDN ablation, with minimal discomfort to the patient and minimal risk of damage to other tissue of the patient.

In some examples, processing circuitry 46 may determine a modality of RDN therapy to apply to patient based on one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.

In some examples, one or more of an amount of power, amplitude, time, and/or cycles of energy delivery may be modulated to obtain effective ablation depths and effective ablation volume, which may be determined by the determined location of ablation. In some examples, rate of change of cycles of RDN therapy may be modulated based on the determined location of the ablation. For example, rate of change of cycles of RDN therapy may be modulated based on body temperature of patient.

In some examples, processing circuitry 46 may receive an indication of a modality of RDN therapy that was selected. For example, a clinician may select a modality of RDN therapy that will be applied, such as using UI 44. In some examples, modality of RDN therapy includes one or more of radiofrequency, cryoablation, ultrasound, microwave, radiation, chemical, nanoparticles, or pulse field ablation.

In some examples, processing circuitry 46 may determine a location of RDN therapy to apply to patient 18 based on the selected modality of RDN therapy and the renal arterial anatomy information. For example, for a patient with a particular renal arterial anatomy information that includes a length of a main renal artery being less than a short main renal artery length threshold (e.g., a short main renal artery) may necessitate a different location for radiofrequency therapy than ultrasound therapy. However, a different patient with different renal arterial anatomy information that includes a length of a main renal artery being greater than a long main renal artery length threshold (e.g., a long main renal artery) may have two more distinct locations for radiofrequency therapy than ultrasound therapy.

In some examples, processing circuitry 46 may determine a location of RDN therapy to apply to patient 18 based on the selected modality of RDN therapy one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.

In some examples, the determined location to apply RDN therapy is a location within a main renal artery of patient 18. In some examples, a desired RDN therapy location may be a location where nerves converge upon the main renal artery. In some examples, renal arterial anatomy information, such as, a length of aorta to hilum, number of branches, location of branches, length of aorta to primary bifurcation point, may indicate where nerves converge upon the main renal artery.

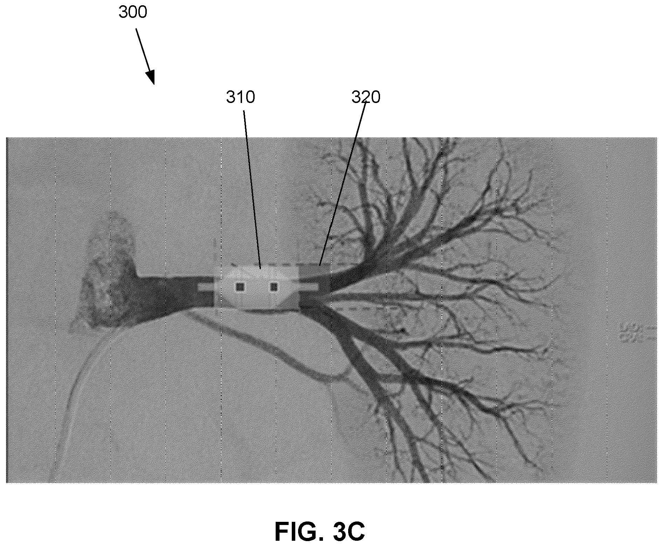

Processing circuitry 46 may output an indication of the determined location to apply RDN therapy to patient 18 and/or the determined modality of RDN therapy to apply to patient 18. For example, computing device 12 may output an indication of the modality of RDN therapy to apply to patient 18 to be displayed on user interface 44 of the computing device 12 to indicate to a clinician the modality of RDN therapy to apply to patient 18. In some examples, processing circuitry 46 may output an indication of the location to apply RDN therapy to apply to patient 18 to be displayed on user interface 44 of the computing device 12 to indicate to a clinician the location to apply RDN therapy to patient 18. FIG. 3C shows an example of an indication of a determined location 310 to apply RDN therapy being displayed on UI 44. In some examples, processing circuitry 46 may output an image 300 of the main renal artery to be displayed on UI 44 that indicates a location to apply RDN therapy 310 and/or a location to avoid applying RDN therapy 320.

In some examples, processing circuitry 46 may output an indication of the determined location to apply RDN therapy to patient 18 and/or the determined modality of RDN therapy to apply to patient 18 to another computing device to indicate to a clinician the location to apply RDN therapy to patient 18 and/or the modality of RDN therapy to apply to patient 18. Accordingly, a clinician may be able to apply a modality of RDN therapy for an individual patient that leads to improved results of RDN therapy and/or apply the RDN therapy at a location for an individual patient that leads to improved results of RDN therapy, which may result more effective and quicker RDN procedures and may result in reduction of unnecessary ablations being applied in RDN procedures. Quicker RDN procedures may require less anesthetic and/or less energy, as well as reducing patient discomfort.

Determining a location and/or modality of RDN therapy to be performed on a particular patient may result in less wasted health care resources, less patient inconvenience and discomfort, less potential unnecessary side effects, etc. This may help reduce unnecessary health care costs and reduce invasive procedures on patients that do not require complex RDN therapy to be applied.

Computing device 12 may be configured to execute an AI engine that operates according to one or more models, such as machine learning models. Machine learning models may include any number of different types of machine learning models, such as neural networks, deep neural networks, dense neural networks, and the like. Although described with respect to machine learning models, the techniques described in this disclosure are also applicable to other types of AI models, including rule-based models, finite state machines, and the like.

Machine learning may generally enable a computing device to analyze input data and identify an action to be performed responsive to the input data. Each machine learning model may be trained using training data that reflects likely input data. The training data may be labeled or unlabeled (meaning that the correct action to be taken based on a sample of training data is explicitly stated or not explicitly stated, respectively).

The training of the machine learning model may be guided (in that a designer, such as a computer programmer, may direct the training to guide the machine learning model to identify the correct action in view of the input data) or unguided (in that the machine learning model is not guided by a designer to identify the correct action in view of the input data). In some instances, the machine learning model is trained through a combination of labeled and unlabeled training data, a combination of guided and unguided training, or possibly combinations thereof. Examples of machine learning include nearest neighbor, naive Bayes, decision trees, linear regression, support vector machines, neural networks, k-Means clustering, Q-learning, temporal difference, deep adversarial networks, evolutionary algorithms or other supervised, unsupervised, semi-supervised, or reinforcement learning algorithms to train one or more models.

Processing circuitry 46 may utilize machine learning, such as a deep learning algorithm or model (e.g., a neural network or deep belief network), to generate (e.g., determine) a score indicative of a location to apply RDN therapy to a patient and/or a modality of RDN therapy to apply to a patient. The generated (e.g., determined) score may be output as an indication of a type of a location to apply RDN therapy to a patient and/or a modality of RDN therapy to apply to a patient.

A ML training device other than computing device 12 may be configured to train the deep learning model to generate a trained model that is then executed on computing device 12. That is, memory 40 may store the trained model, and processing circuitry 46 may execute the trained model.

The ML training device may train a deep learning model to represent a relationship of renal arterial anatomy information and/or other patient metrics of patients to a location to apply RDN therapy to patient 18 and/or a modality of RDN therapy to apply to a patient. In some examples, modality of RDN therapy to apply to a patient may be an input. In other examples, a score indicating a modality of RDN therapy to apply to a patient may be an output. modality of RDN therapy to apply to a patient The ML training device may train a deep learning model to represent a relationship of renal arterial anatomy information, a selected modality of RDN therapy, and/or other patient metrics of patients to a location to apply RDN therapy to a patient. For example, the ML training device may train the deep learning model using imaging data of renal arterial anatomy, renal arterial anatomy information and/or modality of RDN therapy, and/or patient metrics, and responsiveness of a modality and/or location of renal denervation therapy from other patients. In some examples, the ML training device may train the deep leaning model by adjusting the weights of a hidden layer of a neural network model to balance the contribution of each input (e.g., characteristics of the imaging data, renal arterial anatomy information and/or modality of RDN therapy) according to how responsive a patient was to a modality and location of renal denervation therapy.

In some examples, a training set may include renal arterial anatomy information, information of the location of RDN therapy applied, information of the modality of RDN therapy applied, and/or other patient information. In some examples for the training set, a reduction in blood pressure for a patient greater than a first threshold may be considered a good result, a reduction in blood pressure for a patient less than a first threshold but greater than a second threshold may be considered an average result, and a reduction of blood pressure for a patient less than a second threshold may be considered a bad result.

From a training set, a machine learning model may receive patient renal arterial anatomy information and/or other patent information and determine a location and/or modality of RDN therapy that provides optimal results.

In some examples, a modality of RDN therapy may be another input to determine a location to apply RDN therapy that provides optimal results. From a training set, a machine learning model may receive patient renal arterial anatomy information, a selected modality of RDN therapy, and/or other patient information and determine a location to apply RDN therapy that provides optimal results.

Once the deep learning model is trained, computing device 12 may obtain and apply the trained model. For instance, memory 40 may store the trained model for execution by processing circuitry 46 and application on the collected imaging data, renal arterial anatomy information, modality of RDN therapy, and/or a plurality of values representative of respective patient metrics for the patient, to the trained deep learning model. Non-limiting examples of patient metrics may include an age of the patient, a gender of the patient, an ethnic background of the patient, a weight of the patient, a height of the patient, a diet of the patient, and/or other physiological measurements of the patient. The output of the deep learning model may include the score that indicates a location to apply RDN therapy to a patient and/or a modality of RDN therapy to apply to a patient. For example, the score may be a probability that the patient would achieve a target reduction in hypertension in response to a particular modality of RDN therapy being applied at a particular location. In some examples, the score may be indicative of the magnitude of the reduction in systemic blood pressure that the patient may realize after a particular modality of RDN therapy being applied at a particular location. Computing device 12 may display the score to a clinician to aid in determining a particular location to apply RDN therapy to a patient and/or a particular modality of RDN therapy to apply to a patient.

In some examples, computing device 12 may send the collected imaging data, received renal arterial anatomy information and/or selected modality of RDN therapy, to a separate computing system to utilize the separate computing system to perform the machine learning, such as a deep learning algorithm or model, on the received renal arterial anatomy information and/or renal anatomical complexity score.

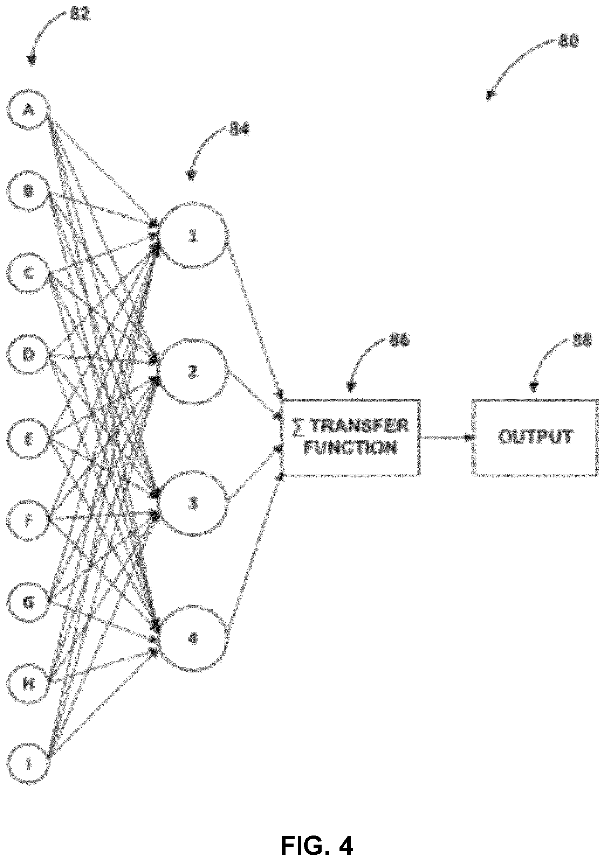

FIG. 4 is a conceptual diagram illustrating an example neural network 80 configured to determine a location to apply RDN therapy to a patient and/or a modality of RDN therapy to apply to a patient that may be diagnosed with hypertension. Neural network 80 is an example of a deep learning model, or deep learning algorithm, trained to generate a score indicative of a location to apply RDN therapy to a patient and/or a modality of RDN therapy to apply to a patient. As discussed above, other types of machine learning and deep learning models or algorithms may be utilized in other examples. Computing device 12 may train, store, and/or utilize neural network 80, but other devices may apply inputs associated with a particular patient to neural network 80 in other examples. Neural network 80 may be stored by computing device 12.

As shown in the example of FIG. 4, neural network 80 comprises three layers. These three layers include input layer 82, hidden layer 84, and output layer 86. Output layer 88 comprises the output from the transfer function of output layer 86. Input layer 82 represents each of the input values A through I provided to neural network 80. The input values may be values for patient metrics such as an age of the patient, a gender of the patient, an ethnic background of the patient, a weight of the patient, a height of the patient, a diet of the patient, and/or other physiological measurements of the patient. The input values may be numerical or categorical as appropriate for each patient metric. In some examples, values for all of these patient metrics may be incorporated into neural network 80.

In addition, some input values of input layer 82 may include one or more characteristics of imaging data, renal arterial anatomy information and/or selected modality of RDN therapy to apply, from the computing device 12. For example, one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery, and/or one or more of radiofrequency, cryoablation, ultrasound, microwave, radiation, chemical, nanoparticles, or pulse field ablation. These characteristics may be input into input layer 82.

Each of the input values for each node in the input layer 82 is provided to each node of hidden layer 84. In the example of FIG. 4, hidden layer 84 include four nodes, but fewer or greater number of nodes may be used in other examples. Each input from input layer 82 is multiplied by a weight and then summed at each node of hidden layer 84. During training of neural network 80, the weights for each input are adjusted to establish the relationship between the renal arterial anatomy information and/or modality of RDN therapy based to efficacy of a location and/or modality of RDN therapy. In some examples, two or more hidden layers may be incorporated into neural network 80, where each layer includes the same or different number of nodes.

The result of each node within hidden layer 84 is applied to the transfer function of output layer 86. The transfer function may be linear or non-linear, depending on the number of layers within neural network 80. Example non-linear transfer functions may be a sigmoid function or a rectifier function. The output 88 of the transfer function may be the score that is generated by computing device 12 in response to applying renal arterial anatomy information and/or modality of RDN therapy for the patient to neural network 80. A deep learning model, such as neural network 80, may enable a computing system such as computing device 12 to determine a location and/or modality of RDN therapy to apply to a patient using a variety of values representing a condition of a particular patient.

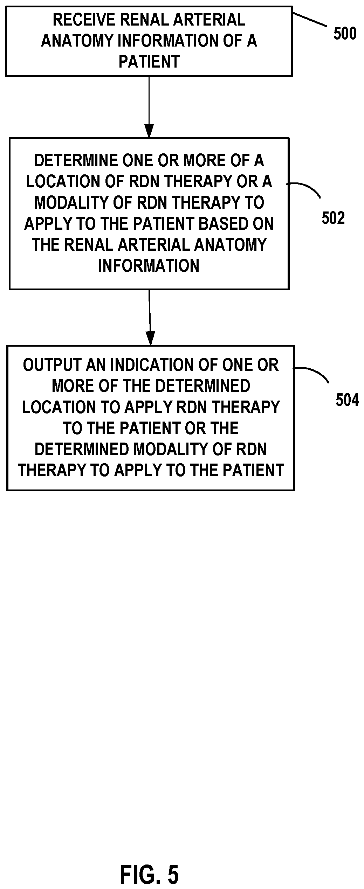

FIG. 5 is a flow diagram illustrating an example technique for operating system 10. As indicated by FIG. 5 processing circuitry 46 may receive renal arterial anatomy information of a patient (500). Renal arterial anatomy information may include one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery. Processing circuitry 46 may determine one or more of a location of RDN therapy or a modality of RDN therapy to apply to the patient based on the renal arterial anatomy information (502). The modality of renal denervation therapy may include one or more of radiofrequency, cryoablation, ultrasound, microwave, radiation, chemical, nanoparticles, or pulse field ablation.

Processing circuitry 46 may determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient. Processing circuitry 46 may output an indication of one or more of the determined location to apply the RDN therapy to the patient or the determined modality of RDN therapy to apply to the patient (504). In some examples, processing circuitry 46 may output an image 300 of the main renal artery to be displayed on UI 44 that indicates a modality of RDN therapy to apply, a location to apply RDN therapy 310, and/or a location to avoid applying RDN therapy 320.

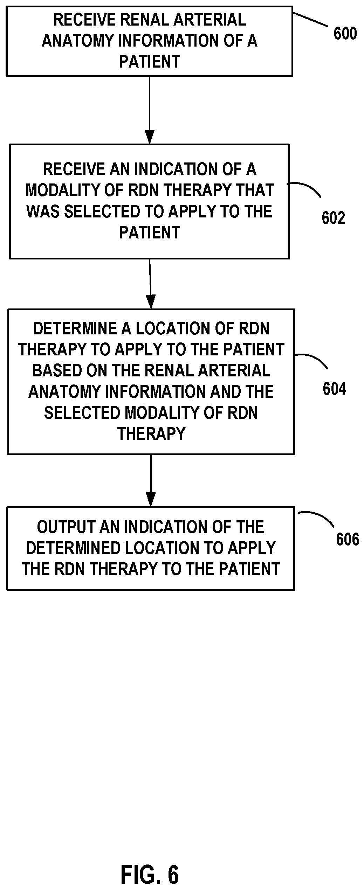

FIG. 6 is a flow diagram illustrating an example technique for operating system 10. As indicated by FIG. 6 processing circuitry 46 may receive renal arterial anatomy information of a patient (600). Renal arterial anatomy information may include one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery. Computing device may receive an indication of a modality of RDN that was selected to apply to the patient (602). The modality of renal denervation therapy may include one or more of radiofrequency, cryoablation, ultrasound, microwave, radiation, chemical, nanoparticles, or pulse field ablation.

Processing circuitry 46 may determine a location of RDN therapy to apply to the patient based on the renal arterial anatomy information and the selected modality of RDN therapy (604). Processing circuitry 46 may determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient. Processing circuitry 46 may output an indication of the determined location to apply the RDN therapy to the patient (606). In some examples, processing circuitry 46 may output an image 300 of the main renal artery to be displayed on UI 44 that indicates a location to apply RDN therapy 310 and/or a location to avoid applying RDN therapy 320.

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure.

Such hardware, software, and firmware may be implemented within the same device or within separate devices to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

Various aspects of the techniques may enable the following examples.

Example 1: A computing device includes a memory; and processing circuitry coupled to the memory, the processing circuitry being configured to: receive renal arterial anatomy information of a patient; determine one or more of a location of renal denervation therapy or a modality of renal denervation therapy to apply to the patient based on the renal arterial anatomy information; and output an indication of one or more of the determined location to apply renal denervation therapy to the patient or the determined modality of renal denervation therapy to apply to the patient.

Example 2: The computing device of example 1, wherein the processing circuitry is further configured to: determine the location of renal denervation therapy to apply to the patient based on the renal arterial anatomy information; and output the indication of the determined location to apply the renal denervation therapy to the patient.

Example 3: The computing device of any of examples 1-2, wherein the processing circuitry is further configured to: receive an indication of the modality of renal denervation therapy that was selected to apply to the patient; determine the location of renal denervation therapy based on the renal arterial anatomy information and the selected modality of renal denervation therapy; and output the indication of the determined location to apply the renal denervation therapy to the patient.

Example 4: The computing device of example 1, wherein the processing circuitry is further configured to: determine the modality of renal denervation therapy to apply to the patient based on the renal arterial anatomy information; and output the indication of the determined modality of renal denervation therapy to apply to the patient.

Example 5: The computing device of any of examples 1-4, wherein the modality of renal denervation therapy includes one or more of radiofrequency, cryoablation, ultrasound, microwave, radiation, chemical, nanoparticles, or pulse field ablation.

Example 6: The computing device of any of examples 1-5, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.

Example 7: The computing device of any one or more of examples 1-6, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.

Example 8: The computing device of example 3, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.

Example 9: A system includes an imaging device configured to collect data indicative of renal arterial anatomy information of a patient; and a computing device communicatively coupled to the imaging device, wherein the computing device comprises: a memory; and processing circuitry coupled to the memory, the processing circuitry being configured to: receive the renal arterial anatomy information of the patient; determine one or more of a location of renal denervation therapy or a modality of renal denervation therapy to apply to the patient based on the renal arterial anatomy information; and output an indication of one or more of the determined location to apply renal denervation therapy to the patient or the determined modality of renal denervation therapy to apply to the patient.

Example 10: The system of example 9, wherein the processing circuitry is further configured to: determine the location of renal denervation therapy to apply to the patient based on the renal arterial anatomy information; and output the indication of the determined location to apply the renal denervation therapy to the patient.

Example 11: The system of any of examples 9-10, wherein the processing circuitry is further configured to: receive an indication of the modality of renal denervation therapy that was selected to apply to the patient; determine the location of renal denervation therapy based on the renal arterial anatomy information and the selected modality of renal denervation therapy; and output the indication of the determined location to apply the renal denervation therapy to the patient.

Example 12: The system of example 1, wherein the processing circuitry is further configured to: determine the modality of renal denervation therapy to apply to the patient based on the renal arterial anatomy information; and output the indication of the determined modality of renal denervation therapy to apply to the patient.

Example 13: The system of any of examples 9-12, wherein the modality of renal denervation therapy includes one or more of radiofrequency, cryoablation, ultrasound, microwave, radiation, chemical, nanoparticles, or pulse field ablation.

Example 14: The system of any of examples 9-13, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.

Example 15: The system of any of examples 9-14, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.

Example 16: The system of example 11, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.

Example 17: A method includes receiving renal arterial anatomy information of a patient; determining one or more of a location of renal denervation therapy or a modality of renal denervation therapy to apply to the patient based on the renal arterial anatomy information; and outputting an indication of one or more of the determined location to apply renal denervation therapy to the patient or the determined modality of renal denervation therapy to apply to the patient.

Example 18: The method of example 17, wherein the method further comprises: determining the location of renal denervation therapy to apply to the patient based on the renal arterial anatomy information; and outputting the indication of the determined location to apply the renal denervation therapy to the patient.

Example 19: The method of any of examples 17-18, wherein the method further comprises: receiving an indication of the modality of renal denervation therapy that was selected to apply to the patient; determining the location of renal denervation therapy based on the renal arterial anatomy information and the selected modality of renal denervation therapy; and outputting the indication of the determined location to apply the renal denervation therapy to the patient.

Example 20: The method of example 17, wherein the method further comprises: determining the modality of renal denervation therapy to apply to the patient based on the renal arterial anatomy information; and outputting the indication of the determined modality of renal denervation therapy to apply to the patient.

Example 21: The method of any of examples 17-20, wherein the modality of renal denervation therapy includes one or more of radiofrequency, cryoablation, ultrasound, microwave, radiation, chemical, nanoparticles, or pulse field ablation.

Example 22: The method of any of examples 17-21, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.

Example 23: The method of any of examples 17-22, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.

Example 24: The method of example 19, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.

Various examples have been described. These and other examples are within the scope of the following claims.

Further disclosed herein is the subject-matter of the following clauses:
1. A computing device comprising:
   a memory; and
   processing circuitry coupled to the memory, the processing circuitry being configured to:
      receive renal arterial anatomy information of a patient;
      determine one or more of a location of renal denervation therapy or a modality of renal denervation therapy to apply to the patient based on the renal arterial anatomy information; and
      output an indication of one or more of the determined location to apply renal denervation therapy to the patient or the determined modality of renal denervation therapy to apply to the patient.
2. The computing device of clause 1, wherein the processing circuitry is further configured to:
   determine the location of renal denervation therapy to apply to the patient based on the renal arterial anatomy information; and
   output the indication of the determined location to apply the renal denervation therapy to the patient.
3. The computing device of any of clauses 1-2, wherein the processing circuitry is further configured to:
   receive an indication of the modality of renal denervation therapy that was selected to apply to the patient;
   determine the location of renal denervation therapy based on the renal arterial anatomy information and the selected modality of renal denervation therapy; and
   output the indication of the determined location to apply the renal denervation therapy to the patient.
4. The computing device of clause 1, wherein the processing circuitry is further configured to:
   determine the modality of renal denervation therapy to apply to the patient based on the renal arterial anatomy information; and
   output the indication of the determined modality of renal denervation therapy to apply to the patient.
5. The computing device of any of clauses 1-4, wherein the modality of renal denervation therapy includes one or more of radiofrequency, cryoablation, ultrasound, microwave, radiation, chemical, nanoparticles, or pulse field ablation.
6. The computing device of any of clauses 1-5, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.
7. The computing device of any one or more of clauses 1-6, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.
8. The computing device of clause 3, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.
9. A system comprising:
   an imaging device configured to collect data indicative of renal arterial anatomy information of a patient; and
   a computing device communicatively coupled to the imaging device, wherein the computing device comprises:
      a memory; and
      processing circuitry coupled to the memory, the processing circuitry being configured to:
         receive the renal arterial anatomy information of the patient;
         determine one or more of a location of renal denervation therapy or a modality of renal denervation therapy to apply to the patient based on the renal arterial anatomy information; and
         output an indication of one or more of the determined location to apply renal denervation therapy to the patient or the determined modality of renal denervation therapy to apply to the patient.
10. The system of clause 9, wherein the processing circuitry is further configured to:
   determine the location of renal denervation therapy to apply to the patient based on the renal arterial anatomy information; and
   output the indication of the determined location to apply the renal denervation therapy to the patient.
11. The system of any of clauses 9-10, wherein the processing circuitry is further configured to:
   receive an indication of the modality of renal denervation therapy that was selected to apply to the patient;
   determine the location of renal denervation therapy based on the renal arterial anatomy information and the selected modality of renal denervation therapy; and
   output the indication of the determined location to apply the renal denervation therapy to the patient.
12. The system of clause 1, wherein the processing circuitry is further configured to:
   determine the modality of renal denervation therapy to apply to the patient based on the renal arterial anatomy information; and
   output the indication of the determined modality of renal denervation therapy to apply to the patient.
13. The system of any of clauses 9-12, wherein the modality of renal denervation therapy includes one or more of radiofrequency, cryoablation, ultrasound, microwave, radiation, chemical, nanoparticles, or pulse field ablation.
14. The system of any of clauses 9-13, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.
15. The system of any of clauses 9-14, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.
16. The system of clause 11, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.
17. A method comprising:
   receiving renal arterial anatomy information of a patient;
   determining one or more of a location of renal denervation therapy or a modality of renal denervation therapy to apply to the patient based on the renal arterial anatomy information; and
   outputting an indication of one or more of the determined location to apply renal denervation therapy to the patient or the determined modality of renal denervation therapy to apply to the patient.
18. The method of clause 17, wherein the method further comprises:
   determining the location of renal denervation therapy to apply to the patient based on the renal arterial anatomy information; and
   outputting the indication of the determined location to apply the renal denervation therapy to the patient.
19. The method of any of clauses 17-18, wherein the method further comprises:
   receiving an indication of the modality of renal denervation therapy that was selected to apply to the patient;
   determining the location of renal denervation therapy based on the renal arterial anatomy information and the selected modality of renal denervation therapy; and
   outputting the indication of the determined location to apply the renal denervation therapy to the patient.
20. The method of clause 17, wherein the method further comprises:
   determining the modality of renal denervation therapy to apply to the patient based on the renal arterial anatomy information; and
   outputting the indication of the determined modality of renal denervation therapy to apply to the patient.
21. The method of any of clauses 17-20, wherein the modality of renal denervation therapy includes one or more of radiofrequency, cryoablation, ultrasound, microwave, radiation, chemical, nanoparticles, or pulse field ablation.
22. The method of any of clauses 17-21, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.
23. The method of any of clauses 17-22, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.
24. The method of clause 19, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.
25. A non-transitory computer-readable storage medium comprising instructions that, when executed by processing circuitry, cause the processing circuitry to:
   receive renal arterial anatomy information of a patient;
   determine one or more of a location of renal denervation therapy or a modality of renal denervation therapy to apply to the patient based on the renal arterial anatomy information; and
   output an indication of one or more of the determined location to apply renal denervation therapy to the patient or the determined modality of renal denervation therapy to apply to the patient.

## Claims

1. A computing device comprising:
a memory; and
processing circuitry coupled to the memory, the processing circuitry being configured to:
receive renal arterial anatomy information of a patient;
determine one or more of a location of renal denervation therapy or a modality of renal denervation therapy to apply to the patient based on the renal arterial anatomy information; and
output an indication of one or more of the determined location to apply renal denervation therapy to the patient or the determined modality of renal denervation therapy to apply to the patient.

2. The computing device of claim 1, wherein the processing circuitry is further configured to:
determine the location of renal denervation therapy to apply to the patient based on the renal arterial anatomy information; and
output the indication of the determined location to apply the renal denervation therapy to the patient.

3. The computing device of any of claims 1-2, wherein the processing circuitry is further configured to:
receive an indication of the modality of renal denervation therapy that was selected to apply to the patient;
determine the location of renal denervation therapy based on the renal arterial anatomy information and the selected modality of renal denervation therapy; and
output the indication of the determined location to apply the renal denervation therapy to the patient.

4. The computing device of claim 1, wherein the processing circuitry is further configured to:
determine the modality of renal denervation therapy to apply to the patient based on the renal arterial anatomy information; and
output the indication of the determined modality of renal denervation therapy to apply to the patient.

5. The computing device of any of claims 1-4, wherein the modality of renal denervation therapy includes one or more of radiofrequency, cryoablation, ultrasound, microwave, radiation, chemical, nanoparticles, or pulse field ablation.

6. The computing device of any of claims 1-5, wherein the renal arterial anatomy information includes one or more of a length of aorta to hilum, number of branches, location of branches, length of aorta to primary bifurcation point, maximum diameter of main renal artery, minimum diameter of the main renal artery, mean diameter of the main renal artery, diameter of branches, length of branches, length of proximal segment of the main renal artery, length of middle segment of the main renal artery, or length of distal segment of the main renal artery.

7. The computing device of any one or more of claims 1-6, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.

8. The computing device of claim 3, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.

9. A system comprising:
an imaging device configured to collect data indicative of renal arterial anatomy information of a patient; and
a computing device communicatively coupled to the imaging device, wherein the computing device comprises:
a memory; and
processing circuitry coupled to the memory, the processing circuitry being configured to:
receive the renal arterial anatomy information of the patient;
determine one or more of a location of renal denervation therapy or a modality of renal denervation therapy to apply to the patient based on the renal arterial anatomy information; and
output an indication of one or more of the determined location to apply renal denervation therapy to the patient or the determined modality of renal denervation therapy to apply to the patient.

10. The system of claim 9, wherein the processing circuitry is further configured to:
determine the location of renal denervation therapy to apply to the patient based on the renal arterial anatomy information; and
output the indication of the determined location to apply the renal denervation therapy to the patient.

11. The system of any of claims 9-10, wherein the processing circuitry is further configured to:
receive an indication of the modality of renal denervation therapy that was selected to apply to the patient;
determine the location of renal denervation therapy based on the renal arterial anatomy information and the selected modality of renal denervation therapy; and
output the indication of the determined location to apply the renal denervation therapy to the patient.

12. The system of claim 1, wherein the processing circuitry is further configured to:
determine the modality of renal denervation therapy to apply to the patient based on the renal arterial anatomy information; and
output the indication of the determined modality of renal denervation therapy to apply to the patient.

13. The system of any of claims 9-12, wherein to determine the one or more of a location of renal denervation therapy or a modality of renal denervation therapy to apply to the patient based on the renal arterial anatomy information, the processing circuitry is configured to apply the renal arterial anatomy information to a machine learning model, the machine learning model trained to generate an output indicating one or more of the determined location to apply renal denervation therapy to the patient or the determined modality of renal denervation therapy to apply to the patient using a training set comprising a plurality of examples of renal arterial anatomy information labeled with a respective one or more of a plurality of locations to apply renal denervation therapy or a plurality of modalities of renal denervation therapy to apply to the patient.

14. The system of claim 11, wherein to determine the location of renal denervation therapy to apply to the patient based on the renal arterial anatomy information and the selected modality of renal denervation therapy, the processing circuitry is configured to apply the renal arterial anatomy information and the selected modality of renal denervation therapy to a machine learning model, the machine learning model trained to generate an output indicating the determined location to apply renal denervation therapy to the patient using a training set comprising a plurality of examples of renal arterial anatomy information and modality of renal denervation therapy labeled with a respective one of a plurality of locations to apply renal denervation therapy to the patient.

15. A non-transitory computer-readable storage medium comprising instructions that, when executed by processing circuitry, cause the processing circuitry to:
receive renal arterial anatomy information of a patient;
determine one or more of a location of renal denervation therapy or a modality of renal denervation therapy to apply to the patient based on the renal arterial anatomy information; and
output an indication of one or more of the determined location to apply renal denervation therapy to the patient or the determined modality of renal denervation therapy to apply to the patient.
